Europäisches Patentamt

**European Patent Office**

Office européen des brevets

(19)

(11) Numéro de publication: **0 177 411 B1**

(12) **FASCICULE DE BREVET EUROPEEN**

(45) Date de publication du fascicule du brevet: **01.08.90**

(51) Int. Cl.⁵: **B 01 D 15/08** // C12N9/00

(21) Numéro de dépôt: **85401872.8**

(22) Date de dépôt: **25.09.85**

(54) **Nouveau complexe d'élution notamment pour la chromatographie d'affinité et la précipitation par affinité.**

(30) Priorité: **26.09.84 FR 8414786**

(43) Date de publication de la demande:
**09.04.86 Bulletin 86/15**

(45) Mention de la délivrance du brevet:
**01.08.90 Bulletin 90/31**

(84) Etats contractants désignés:
**AT BE CH DE FR GB IT LI LU NL SE**

(56) Documents cités:
**EP-A-0 064 378**
**EP-A-0 064 833**

**JOURNAL OF CHROMATOGRAPHY, vol. 216, 30 octobre 1981, pages 175-190, Elsevier Scientific Publishing Co., Amsterdam, NL; D.A.P. SMALL et al.: "High-performance liquid affinity chromatrography of enzymes on silica-immobilised triazine dyes"**
**CHEMISTRY AND INDUSTRY, no. 20, 17 octobre 1981, pages 726-732, London, GB; Y. HEY et al.: "Dyes - a colourful addition to protein purification"**

(73) Titulaire: **Etablissement Public dit: CENTRE NATIONAL DE LA RECHERCHE SCIENTIFIQUE (CNRS)**
**15, Quai Anatole France**
**F-75007 Paris (FR)**

(72) Inventeur: **Vijayalakshmi, Mookambeswaran Arunachalam**
**Les Tulipes 11 Square du Commandant Fournaise**
**F-60200 Compiègne (FR)**
Inventeur: **Rajgopal, Sunanda**
**4 rue Pierre et Marie Curie Appartement 38**
**F-60200 Compiègne (FR)**

(74) Mandataire: **Corre, André et al**
**Cabinet Corre A. 17, rue Pasteur**
**F-92300 Levallois (FR)**

(56) Documents cités:
**Journal of Chromatography Library, Vol. 12 (1978), Elsevier Scientific Publishing Co., pp. 62,63,234,235,327,328**
**European Journal of Biochemistry, Vol. 41(1974), pp. 353-357**

## EP 0 177 411 B1

(56) Documents cités:

The Biochemical Journal, Vol. 153 (1976), pp.
153, 351-361
Biochimica et Biophysica Acta, 385(1975), pp.
20-27

**Description**

La présente invention est relative à un nouveau complexe d'élution particulièrement adapté à la chromatographie d'affinité et à la précipitation par affinité.

La chromatographie d'affinité est une technique actuellement très utilisée pour isoler des enzymes et les purifier de façon à obtenir l'enzyme recherchée avec un grand degré de pureté. Une des phases délicates de cette technique est l'élution: il faut exclusivement entraîner l'enzyme souhaitée ou parfois un ensemble d'enzymes. En effet plusieurs types d'enzymes ont pu être retenus sur la colonne de chromatographie. Afin d'être assuré que seule l'enzyme recherchée est éluée, on utilise une solution contenant le substrat de cette enzyme.

Ainsi, par exemple, les hexokinases qui ont pour substrat le nicotinamide-adénine-dinucléotide phosphate (NADP), sont éluées au moyen d'une solution renfermant ce composé; de même, la luciférase qui a pour substrat spécifique l'adénosine-triphosphate (ATP), est éluée au moyen d'une solution contenant cet acide.

La précipitation par affinité est une autre technique utilisée depuis une date récente pour isoler et purifier des enzymes. Cette technique, étroitement liée à la chromatographie par affinité et à l'immuno-précipitation, comporte deux étapes. La première étape consiste à précipiter l'enzyme à purifier a l'aide d'un ligand bi-fonctionnel sous forme de dimère. Au cours de la deuxième étape, on dissocie de façon spécifique cette enzyme au moyen d'une solution contenant son substrat.

Mais les substrats, en particulier ceux énoncés ci-dessus, sont parfois coûteux, car d'obtention difficile. De plus, une fois l'enzyme extraite de la solution d'élution, on ne peut réutiliser la solution restante, cette impossibilité de recyclage augmente encore le prix de revient de l'enzyme.

L'état de la technique concernant la chromatographie d'affinité peut être illustré par les documents suivants:

Journal of Chromatography, 216 (1981) pages 175 à 190: "High-performance liquid affinity chromatography of enzymes on silica-immobilised triazine dyes."

EP—A—0 064 378: chromatographie d'affinité utilisant des ions métalliques.

EP—A—0 064 833: chromatographie d'affinité liquide à haute pression.

Chemistry and Industry no 20, 17 Octobre 1981, pages 726 à 732: "Dyes, a colourful addition to protein purification".

Journal of Chromatography Library, vol. 12, Elsevier. 1978, pages 62, 63, 234, 235, 327 et 328.

European Journal of Biochemistry, vol. 41, no 2, 1974, pages 353 à 357 "Affinity chromatography on immobilised Adenosine 5'-monophosphate".

The Biochemical Journal, (1976) 153, pages 351 à 361: "Studies on the use of Sepharose-N-(6-Amino-hexanoyl)-2-Amino-2-Deoxy-D-Glucopyranose for the Large-Scale Purification of Hepatic Glucokinase."

Biochimica et Biphysica Acta, 385 (1975) page 20 à 27: "Dynamic affinity chromatography of heavy Méromyosin Subfragment-1."

Aussi un des buts de la présente invention estil de fournir un complexe d'élution notamment pour chromatographie d'affinité et précipitation par affinité aussi performant que le substrat spécifique pour éluer l'enzyme retenue sur la colonne de chromatographie.

Un objet de l'invention est un tel complexe d'élution dont la production est facile et peu coûteuse permettant ainsi une production d'enzyme à un prix de revient aussi bas que possible.

Ce but et cet objet, ainsi que d'autres qui apparaîtront par la suite, sont atteints par un complexe d'élution notamment pour chromatographie d'affinité et la précipitation par affinité qui, selon la présente invention, comprend un sel de magnésium, de l'acide éthylène-diamino-tétracétique (EDTA), une sucre différent du glucose et a un pH légèrement basique.

Avantageusement le sel de magnésium dont la concentration est comprise entre 1 mM et 30 mM, est du sulfate de magnésium ($MgSO_4$).

De préférence, la concentration en acide étylène-diamino-tétracétique est comprise entre 30 mM et 50 mM.

Selon un mode préféré de réalisation de l'invention, le sucre de ce complexe d'élution est du ribose ou du mannose.

Avantageusement, la concentration en ribose ou en mannose dans le complexe est de 5 mM.

De préférence, ce complexe comprend également du dithiothréitol à une concentration de l'ordre de 1 mM.

Avantageusement, le complexe d'élution selon la présente invention comprend 20 mM de sulfate de magnésium, 40 mM d'EDTA, 5 mM de ribose et 1 mM de dithiothréitol.

Selon un autre mode de réalisation, ce complexe d'élution comprend 5 mM de sulfate de magnésium, 40 mM d'EDTA et 5 mM de ribose.

Ainsi qu'il a été dit précédemment, la présente invention est relative à un complexe d'élution pour la chromatographie d'affinité et la précipitation par affinité qui permet d'éluer une enzyme sans utiliser le substrat de cette enzyme.

La Demanderesse a été conduite à étudier un complexe siimple, puovant être produit industriellement, de bonne conservation et peu coûteux. Ce complexe comprend notamment du magnésium sous forme de sel, de l'acide éthylène-diamino-tétracétique (EDTA) et un sucre tel que du ribose ou du mannose, auquel

# EP 0 177 411 B1

on peut ajouter du dithothréitol (DTT). Le pH de ce complexe doit être légèrement basique: en général il est de l'ordre de 7,4.

Plus précisément ce complexe comprend de 1 mM à 20 mM d'ions magnésium, de 30 à 50 mM d'acide éthylène-diamino-tétracétique et environ 5 mM de sucre, avec éventuellement environ 1 mM de dithiothréitol.

Les exemples suivants de réalisation de la présente invention, qui n'ont aucun caractère limitatif, permettent à l'homme du métier de mieux comprendre la présente invention ainsi que ses avantages.

## Exemple 1
### Récupération de la luciférase par chromatographie d'affinité

La luciférase est une enzyme dont le substrat spécifique est l'adénosine-triphosphate ou A.T.P. Cette enzyme est isolée par chromatographie d'affinité sur une colonne contenant du Blue-Sépharose®.

L'élution permettant de récupérer la luciférase a été réalisée avec plusieurs éluants dont la composition est indiqué dans le tableau I ci-après:

### TABLEAU I

| Eluant | 1 | 2 | 3 | 4 | 5 |
|---|---|---|---|---|---|
| [1]MOPS | 0,01 mM | — | — | — | — |
| ATP | 0,5 mM | — | — | — | — |
| EDTA | — | 30 mM | 40 mM | 40 mM | 40 mM |
| [2]Mg$^{++}$ | 20 mM | 20 mM | 20 mM | 20 mM | 20 mM |
| Ribose | — | — | — | 5 mM | — |
| Mannose | — | — | — | — | 5 mM |
| Glucose | — | — | 5 mM | — | — |
| DTT | 1 mM | — | 1 mM | 1 mM | 1 mM |

[1] MOPS: acide 3(N-morpholino)-propane sulfonique
[2] Sous forme de sulfate de magnésium MgSO$_4$

Dans le tableau II ci-dessous sont regroupés les résultats des élutions réalisées avec les éluants mentionnés dans le tableau I.

### TABLEAU II

| Eluant | pH | % d'activité de luciférase | % de protéine récupérée | multiplication de purification |
|---|---|---|---|---|
| 1 | 7,4 | 180 | 63 | 3,0 |
| 2 | 7,4 | 20 | 70 | — |
| 3 | 7,4 | Nul | Négligeable | — |
| 4 | 7,4 | 120 | 58 | 2,0 |
| 5 | 7,4 | 67 | 37 | 1,8 |

Il apparaît donc qu'un complexe comprenant un sel de magnésium, de l'acide éthylène-diamino-tétracétique (EDTA) et un sucre qui ne peut être du glucose, mais par exemple du ribose ou du mannose, ainsi que éventuellement du dithiothréitol, peut remplacer advantageusement l'ATP pour éluer la luciférase. Ainsi, les éluants 4 ou 5 sont satisfaisants, l'éluant 4 étant le plus performant.

## Exemple 2
### Récupération de l'hexokinase par chromatographie d'affinité

4

L'hexokinase est une enzyme dont le substrat spécifique est le nicotinamide-adénine-dinucléotide phosphate (NADP). Cette enzyme est isolée par chromatographie d'affinité sur une colonne de Sépharose 4B® liée à du Procion Blue HB®.

L'éluant permettant de récupérer l'hexokinase pure retenue sur une telle colonne est usuellement une solution de NADP. Selon l'invention, la Demanderesse a mis en évidence qu'un complexe comprenant 5 mM de sel de magnésium tel que du sulfate de magnésium ($MgSO_4$), et 40 mM d'EDTA, auquel on ajouté 5 mM de ribose, convient parfaitement pour récupérer toute l'hexokinase retenue par une telle colonne. Le pH de ce complexe est de l'ordre de 7,4.

Exemple 3

Récupération de la lactate déshydrogénase par chromatographie d'affinité

La lactate déshydrogénase est une enzyme dont un des substrats spécifique est le $NAD^+$ (nicotinamide-adénine-dinucléotide). Cette enzyme est isolée par chromatographie d'affinité sur une colonne de Sépharoe 4B® couplée à du Procion Blue HB®.

L'éluant permettant de récupérer la lactate déshydrogénase pure retenue sur une telle colonne est usuellement une solution de $NAD^+$ et de pyruvate. Selon l'invention, la Demanderesse a mis en évidence qu'un complexe comprenant 20 mM de sel de magnésium tel que du sulfate de magnésium ($MgSO_4$) et 40 mM d'EDTA auquel on ajoute 5 mM de ribose, avec ou sans addition de pyruvate, convient parfaitement pour récupérer toute la lactate déshydrogénase retenue par une telle colonne. Le pH de ce complexe est de l'ordre de 7,5.

Exemple 4

Récupération de la lactate déshydrogénase par précipitation par affinité

Cette enzyme est précipitée à l'aide du dimère de Cibacron Blue®.

L'éluant permettant de récupérer la plus grande partie (90%) de la déshydrogénase pure précipitée est habituellement une solution de $NAD^+$ et de pyruvate. Selon l'invention la Demanderesse a mis en évidence qu'un complexe comprenant 20 à 30 mM de sel de magnésium tel que sulfate de magnésium et 40 mM d'EDTA auquel on ajoute 5 mM de ribose, avec ou sans addition de pyruvate, convient parfaitement pour récupérer la même quantité (90%) de l'enzyme. Le pH de ce complexe est voisin de 7,5.

**Revendications**

1. Complexe d'élution notamment pour chromatographie d'affinité et précipitation par affinité, caractérisé en ce qu'il comprend un sel de magnésium, de l'acide éthylène-diamino-tétracétique (EDTA) et un sucre différent du glucose, et en ce qu'il a un pH légèrement basique.

2. Complexe selon la revendication 1, caractérisé en ce que la concentration en sel de magnésium est comprise entre 1 mM et 30 mM.

3. Complexe selon la revendication 2, caractérisé en ce que le sel de magnésium est du sulfate de magnésium ($MgSO_4$).

4. Complexe selon l'une quelconque des revendications 1 à 3, caractérisé en ce que la concentration en acide éthylène-diamino-tétracétique est comprise entre 30 mM et 50 mM.

5. Complexe selon la revendication 1, caractérisé en ce que le sucre est le ribose ou le mannose.

6. Complexe selon la revendication 5, caractérisé en ce que la concentration en ribose ou en mannose est de l'ordre de 5 mM.

7. Complexe selon l'une quelconque des revendications 1 à 6, caractérisé en ce qu'il comprend également du dithiothréitol.

8. Complexe selon la revendication 7, caractérisé en ce que la concentration en dithiothréitol est de l'ordre de 1 mM.

9. Complexe selon l'une quelconque des revendications 1 à 8, caractérisé en ce qu'il comprend 20 mM de sulfate de magnésium, 40 mM d'EDTA, 5 mM de ribose et 1 mM de dithiothréitol.

10. Complexe selon l'une quelconque des revendications 1 à 6, caractérisé en ce qu'il comprend 5 mM de sulfate de magnésium, 40 mM d'EDTA et 5 mM de ribose.

**Patentansprüche**

1. Elutionskomplex, insbesondere für die Affinitätschromatographie und die Präzipitation aufgrund von Affinität, dadurch gekennzeichnet, daß er ein Magnesiumsalz, Ethylendiaminotetraessigsäure (EDTA) und einen von Glucose abweichenden Zucker enthält und daß er einen leicht basischen pH-Wert aufweist.

2. Komplex nach Anspruch 1, dadurch gekennzeichnet, daß die Konzentration des Magnesiumsalzes zwischen 1 und 30 millimolar liegt.

3. Komplex nach Anspruch 2, dadurch gekennzeichnet, daß es sich beim Magnesiumsalz um Magnesiumsulfat ($MgSO_4$) handelt.

4. Komplex nach einem der Ansprüche 1 bis 3, dadurch gekennzeichnet, daß die Konzentration der Ethylendiaminotetraessigsäure zwischen 30 und 50 millimolar liegt.

5. Komplex nach Anspruch 1, dadurch gekennzeichnet, daß es sich beim Zucker um Ribose oder Mannose handelt.

6. Komplex nach Anspruch 5, dadurch gekennzeichnet, daß die Konzentration an Ribose und Mannose in der Größenordnung von 5 millimolar liegt.

7. Komplex nach einem der Ansprüche 1 bis 6, dadurch gekennzeichnet, daß er auch Dithiothreit enthält.

8. Komplex nach Anspruch 7, dadurch gekennzeichnet, daß die Konzentration an Dithiothreit in der Größenordnung von 1 millimolar liegt.

9. Komplex nach einem der Ansprüche 1 bis 8, dadurch gekennzeichnet, daß er 20 millimolar an Magnesiumsulfat, 40 millimolar an EDTA, 5 millimolar an Ribose und 1 millimolar an Dithiothreit umfaßt.

10. Komplex nach einem der Ansprüche 1 bis 6, dadurch gekennzeichnet, daß er 5 millimolar an Magnesiumsulfat, 40 millimolar an EDTA und 5 millimolar an Ribose umfaßt.

**Claims**

1. An elution complex especially for affinity chromatography and affinity precipitation, characterised in that it comprises a magnesium salt, ethylene diamino tetra-acetic acid (EDTA) and a sugar other than glucose, and in that it has a slightly basic pH value.

2. A complex according to claim 1, characterised in that the concentration of magnesium salt is between 1 mM and 30 mM.

3. A complex according to claim 2, characterised in that the magnesium salt is magnesium sulphate $(MgSO_4)$.

4. A complex according to any one of claims 1 to 3, characterised in that the concentration of ethylene diamino tetra-acetic acid is between 30 mM and 50 mM.

5. A complex according to claim 1, characterised in that the sugar is ribose or mannose.

6. A complex according to claim 5, characterised in that the concentration of ribose or mannose is of the order of 5 mM.

7. A complex according to any one of claims 1 to 6, characterised in that it also comprises dithiothreitol.

8. A complex according to claim 7, characterised in that the concentration of dithiothreitol is of the order of 1 mM.

9. A complex according to any one of claims 1 to 8, characterised in that it comprises 20 mM of magnesium sulphate, 40 mM of EDTA, 5 mM of ribose and 1 mM of dithiothreitol.

10. A complex according to any one of claims 1 to 6, characterised in that it comprises 5 mm of magnesium sulphate, 40 mM of EDTA and 5 mM of ribose.